# EUROPÄISCHE PATENTSCHRIFT

(11) **EP 2 265 572 B1**
(45) Veröffentlichungstag und Bekanntmachung des Hinweises auf die Patenterteilung: **18.07.2012**
(21) Anmeldenummer: 09721321.9
(22) Anmeldetag: 18.03.2009
(51) Int. Cl.: C07C 271/28, C07C 269/06

(54) **METALLCARBAMATE AUS DIAMINOPHENYLMETHAN**
METAL CARBAMATES FORMED FROM DIAMINOPHENYLMETHANE
CARBAMATES MÉTALLIQUES À BASE DE DIAMINOPHÉNYLMÉTHANE

(30) Priorität: 18.03.2008 EP 08152934
(43) Veröffentlichungstag der Anmeldung: 29.12.2010
(73) Patentinhaber: BASF SE, 67056 Ludwigshafen (DE)
(72) Erfinder: LEITNER, Andreas, RAINBACH I N 4281 (AT); BAUMANN, Robert, 68529 Mannheim (DE); SIEGEL, Wolfgang, 67117 Limburgerhof (DE)
(86) Internationale Anmeldenummer: PCT/EP2009/053168
(87) Internationale Veröffentlichungsnummer: WO 2009/115537

(56) Entgegenhaltungen:
- DE-A1- 3 202 690
- TUNDO, PIETRO ET AL: "Dimethyl Carbonate as an Ambident Electrophile" JOURNAL OF ORGANIC CHEMISTRY ( 2005 ), 70(6), 2219 -2224 CODEN: JOCEAH; ISSN: 0022-3263, 2005, XP002526970 in der Anmeldung erwähnt
- ARESTA, MICHELE ET AL: "Reaction of aromatic diamines with diphenyl carbonate catalyzed by phosphorus acids: a new clean synthetic route to mono- and dicarbamates" TETRAHEDRON , 54(46), 14145-14156 CODEN: TETRAB; ISSN: 0040-4020, 1998, XP002526971

## Beschreibung

Gegenstand der Erfindung sind Metallcarbamate aus Diaminophenylmethan (MDA) und ein Verfahren zu Ihrer Herstellung.

Carbamate sowie ihre Herstellung und Verwendung sind bekannt.

Zur Herstellung von Carbamaten und Urethanen ist eine Reihe von Verfahren bekannt.

Dabei werden beispielsweise Lewissäuren, wie z.B. Uransalze (US 3,763,217), Aluminiumspäne mit lod und Hg Promotoren (US 4,550,188), Zink-, Eisen-, Antimon- und Zinnsalze (US 4,268,683, 4,268,684, EP 391473) als Katalysatoren verwendet. Nachteilig für den industriellen Einsatz dieser Verfahren sind die zum Teil niedrigen Umsätze, niedrigen Selektivitäten oder beides.

Hohe Selektivitäten und Ausbeuten werden beispielsweise bei lewissäurekatalysierten Verfahren (Pb-Salze als Katalysatoren) erhalten, wenn ein hoher Überschuss an Dialkylcarbonat (Amin:Carbonat 1:20) eingesetzt wird (WO 98/55451, WO 98/56758). Der hohe Überschuss an Dialkylcarbonat führt zu großen Rückführströmen.

In anderen Fällen können hohe Ausbeuten an Urethan erreicht werden, wenn der in der Urethanisierung entstandene Harnstoff in einer zusätzlichen Reaktion thermisch in das entsprechende Urethan rückgespalten wird (EP 048371 (Katalysatoren: Blei-, Titan-, Zink- und Zirconsalze), EP 391473 (Katalysator: Zn-Salze). Die Rückspaltung erfordert einen zusätzlichen, energetisch aufwändigen Schritt.

Ein weiterer Nachteil bei der Anwendung von Lewissäuren als Homogenkatalysatoren sind die im Produkt verbleibenden Katalysatorrückstände, die nur unvollständig abgetrennt werden können.

In WO 2007/015852 wird der Einsatz von lewissauren Heterogenkatalysatoren für die Urethanisierung von aromatischen Aminen beschrieben. Dadurch entfällt eine aufwändige Abtrennung eines Homogenkatalysators. Die erhaltenen Umsätze sind für großtechnische Anwendungen zu niedrig und nehmen gemeinsam mit der Selektivität mit zunehmender Standzeit des heterogenen Katalysators ab.

Es ist weiterhin bekannt, Urethane aus aromatischen Aminen unter Verwendung von basischen Verbindungen, beispielsweise Alkalimetall- oder Erdalkalimetallalkoholaten herzustellen.

In DE 3202690 wird die Herstellung von aromatischen Urethanen durch Reaktion von Anilin und Dialkylcarbonaten in Gegenwart einer geringen Menge eines Metallalkoholates als Katalysator beschrieben. Die in den Beispielen beschriebenen Umsätze sind unvollständig und die erzielten Selektivitäten für eine industrielle Anwendung nicht ausreichend.

In Journal of Organic Chemistry, 2005, 70, 2219-2224 wird die Umsetzung von Anilin mit einem großen Überschuss an Dimethylcarbonat (40 facher Überschuss) in Gegenwart eines Überschusses an Base wie Natriummethylat (NaOMe) oder Kaliumtertiärbutylat (KOtBu) beschrieben. Mit NaOMe wurde eine Selektivität von 67% nach einer Reaktionszeit von 210min erhalten. Mit KOtBu wird nach 1 min eine Selektivität von 100% beschrieben, die jedoch mit zunehmender Reaktionszeit auf 60% durch Bildung des Nebenproduktes N-Methylcarbanilat abfällt. Umsätze und isolierte Ausbeuten wurden nicht beschrieben.

N-Arylcarbamate können zu Isocyanaten umgesetzt werden. Derartige Verfahren sind allgemein bekannt. Durch diese Verfahrensweise können auf phosgenfreiem Wege Diisocyanate hergestellt werden. Nach derartigen Verfahren werden insbesondere aliphatische Diisocyanate hergestellt.

Bei aromatischen Diisocyanaten ist die Herstellung nach einem phosgenfreien Verfahren schwierig, da auf Grund der hohen Reaktivität der aromatischen Verbindungen eine Reihe von Nebenreaktionen ablaufen. Es wäre jedoch wünschenswert, wenn auch aromatische Diisocyanate, die technisch eine große Bedeutung haben, durch phosgenfreie Verfahren hergestellt werden könnten.

Es war die Aufgabe der vorliegenden Erfindung, eine einfache Möglichkeit zu finden, Ausgangsprodukte für die Herstellung von aromatischen Diisocyanaten nach einem phosgenfreien Verfahren bereitzustellen, die mit einer hohe Selektivität, einer hohen Ausbeute und mit hoher Reinheit hergestellt werden können.

Es wurde überraschenderweise gefunden, dass es möglich ist, Metallcarbamate auf Basis von Diaminophenylmethan (MDA) in reiner Form zu isolieren. Diese können nach Umsetzung mit protischen Verbindungen, insbesondere mit Alkoholen oder bevorzugt mit Wasser, zum entsprechenden Diurethan (MDU) und in einem Folgeschritt durch thermische Spaltung zu MDI (Methylendiphenyldiisocyanät)umgesetzt werden.

Gegenstand der Erfindung sind demzufolge Metallcarbamate der allgemeinen Formel (I) 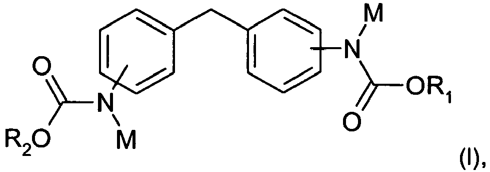 wobei R1 und R2 gleich oder verschieden, eine Alkylgruppe mit 1-18 Kohlenstoffatomen und M ein Alkalimetallatom bedeuten.

Besonders bevorzugt sind Alkylgruppen mit 2-7 Kohlenstoffatomen, die verzweigt, unverzweigt oder cyclisch, insbesondere verzweigt oder unverzweigt sein können.

In einer bevorzugten Ausführungsform der Erfindung sind die Gruppen R1 und R2 gleich.

Gegenstand der Erfindung ist weiterhin ein Verfahren zur Herstellung von Metallcarbamaten der allgemeinen Formel (I) durch Umsetzung von
a) Tolylendiamin mit
b) einem Alkylcarbonat der allgemeinen Formel (II), 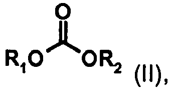 wobei R1 und R2 die oben genannte Bedeutung hat, und
c) einer Metallverbindung der allgemeinen Formel (III)

   M(R3)ₙ

   wobei
   - M: ein Alkalimetallmetallatom bedeutet,
   - R3: gleich R1 und R2, ein Amid oder ein Alkylsilazid sind und
   - n: gleich 1 ist.

In einer Ausführungsform der Erfindung ist die Alkylkette R1 und/oder R2 mit Heteroatomen modifiziert. Bei den Heteroatomen kann es sich um Halogenatome handeln, vorzugsweise um Fluoratome und/oder Chloratome, besonders bevorzugt um Fluoratome. In einer anderen Ausführungsform handelt es sich bei den Heteroatomen um Sauerstoffatome. Diese liegen bevorzugt als Ethergruppen vor. Es hat sich gezeigt, dass sich die Urethane, die unter Verwendung von Dialkylcarbonaten mit Heteroatomen in der Alkylgruppe hergestellt wurden, besonders leicht zu Isocyanaten spalten lassen.

Bevorzugt ist R1 und/oder R2 ein Ethyl-, Propyl, Butyl, Di-2-Methylpropyl-, Di-2-Methylpropyl-, Di-3-Methylbutyl-, Di-n-Pentyl-, 2-methoxyethyl-, 2-ethoxyethyl- oder eine 2,2,2-Trifluorethylgruppe.

Besonders bevorzugt sind R1 und R2 gleich. Das hat den Vorteil, dass bei der Herstellung der erfindungsgemäßen Produkte (I) und bei einer eventuellen Weiterverarbeitung zu Urethanen und deren Umsetzung zu Isocyanaten weniger Produkte im Verfahren sind.

Die Verbindungen der allgemeinen Formel (I) sind bei Raumtemperatur fest und können problemlos und in hoher Reinheit aus der Reaktionslösung abgetrennt werden. Sofern erforderlich, können sie in einem weiteren Verfahrensschritt gereinigt werden.

Die Herstellung der Verbindungen der allgemeinen Formel (I) erfolgt, wie oben beschrieben, durch Umsetzung der Komponenten a), b) und c).

Als Diaminophenylmethan (MDA) können sämtliche Isomere in beliebigen Mischungsverhältnissen eingesetzt werden. Vorzugsweise kommen 2,4'-diaminophenylmethane, 4,4'-diaminophenylmethan, 2,2'-diaminophenylmethan und höhere Homologe (Polyaminopolyphenylmethane) und Isomerengemische zum Einsatz.

In einer bevorzugten Ausführungsform der Erfindung sind die Dialkycarbonate b) ausgewählt aus der Gruppe, enthaltend Diethylcarbonat_{;} Di-n-Propylcarbonat, Di-n-butylcarbonat, Di-2-Methylpropylcarbonat, Di-3-Methylbutylcarbonat, Di-n-Pentylcarbonat, Bis-2-methoxyethylcarbonat, Bis-2-ethoxyethylcarbonat, Bis-2,2,2-Trifluorethylcarbonat.

Bei der Metallverbindung c) handelt es sich vorzugsweise um basische organische Metallverbindungen, insbesondere um Verbindungen von Alkalimetallen. Dabei kann es sich beispielsweise um Stickstoffatome enthaltende Verbindungen, beispielsweise Amide, wie Natriumamid oder Siliziumatome und Stickstoffatome enthaltende Verbindungen, beispielsweise Lithiumhexamethyldisilazid, handeln.

Besonders bevorzugt handelt es sich bei der Base um die Alkoholate von Alkalimetallen.

Das Alkalimetall M ist vorzugsweise Lithium, Natrium oder Kalium. Die Alkoholreste entsprechen vorzugsweise denen der eingesetzten Alkylcarbonate der allgemeinen Formel (II).

Die Herstellung der Verbindungen der allgemeinen Formel (I) erfolgt vorzugsweise unter Normaldruck bei Temperaturen zwischen 100 und 150°C. Die Ausbeute des Verfahrens liegt zwischen 95-100 %.

Bei der Umsetzung beträgt das Verhältnis von Carbonatgruppen zu Aminogruppen von 1: 1 bis 10:1, besonders bevorzugt von 2:1 bis 3:1.

Die Metallverbindung c) wird bevorzugt in stöchiometrischer Menge, besonders bevorzugt in einem molaren Verhältnis 1:1, bezogen auf die Aminogruppen, das heißt in einem Verhältnis von etwa einem Mol Base pro Aminogruppe, eingesetzt.

Die erfindungsgemäßen Metallcarbamate können, wie beschrieben, durch Reprotonierung mit Wasser zu reinem MDU umgesetzt werden.

Dass durch ein einfaches Verfahren reine Metallcarbamate hergestellt werden konnten und somit die Aufgabe der Erfindung gelöst werden konnte, war für den Fachmann nicht vorhersehbar.

Es war auch nicht nötig, mit einem hohen Überschuss der Komponente b) zu arbeiten. Trotz der unterschiedlichen Reaktivität der beiden Aminogruppen des MDA kam es zu einer gleichmäßigen Umsetzung der beiden Aminogruppen.

## Patentansprüche

1. Metallcarbamate der allgemeinen Formel (I) 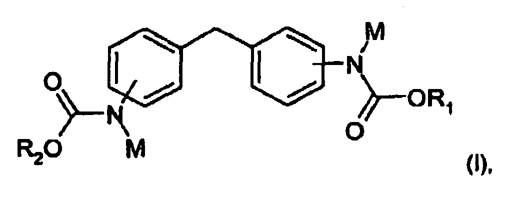 wobei R₁ und R₂, gleich oder verschieden, eine Alkylgruppe mit 1 bis 18 Kohlenstoffatomen, und M ein Alkalimetallion bedeuten.

2. Metallcarbamate nach Anspruch 1, **dadurch gekennzeichnet, dass** die Alkylgruppen R₁ und R₂ 2-18 Kohlenstoffatome in der Kette enthalten.

3. Metallcarbamate nach Anspruch 1, **dadurch gekennzeichnet, dass** die Alkylgruppen R₁ und R₂ 2-7 Kohlenstoffatome in der Kette enthalten.

4. Metallcarbamate nach Anspruch 1, **dadurch gekennzeichnet, dass** die Alkylgruppen R₁ und R₂ ausgewählt sind aus der Gruppe, enthaltend eine Ethyl-, Propyl, Butyl, Di-2-Methylpropyl-, Di-2-Methylpropyl-, Di-3-Methylbutyl-, Di-n-Pentyl-, 2-methoxyethyl-, 2-ethoxyethyl- oder eine 2,2,2-Trifluorethylgruppe.

5. Metallcarbamate nach Anspruch 1, **dadurch gekennzeichnet, dass** die Alkylgruppen Heteroatome enthalten.

6. Metallcarbamate nach Anspruch 1, **dadurch gekennzeichnet, dass** die Alkylgruppen Sauerstoffatome enthalten.

7. Verfahren zur Herstellung von Metallcarbamaten der allgemeinen Formel (I) durch Umsetzung von
a) Tolylendiamin mit
b) einem Alkylcarbonat der allgemeinen Formel (II). 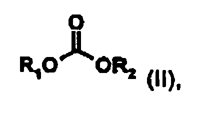 wobei R₁ und R₂ die oben genannte Bedeutung haben, und
c) einer Metallverbindung der allgemeinen Formel (III)
M(R₃)ₙ
wobei
M ein Alkalimetallmetall bedeutet,
R₃ gleich R₁ und R₂ oder ein Amid oder ein Alkylsilazid ist und
n gleich 1 ist.

8. Verfahren zur Herstellung von Methylendiphenyldiurethan, **dadurch gekennzeichnet, dass** eine Verbindung der allgemeinen Formel (I) mit Wasser umgesetzt wird.

## Claims

1. A metal carbamate of the general formula (I) 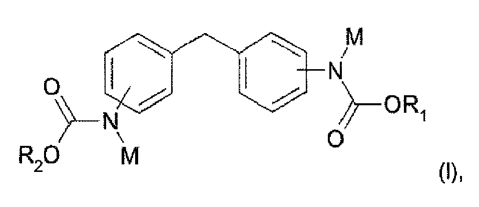 where R₁ and R₂ are the same or different and are each an alkyl group having 1 to 18 carbon atoms and M is an alkali metal ion

2. A metal carbamate according to claim 1, wherein the alkyl groups R₁ and R₂ each comprise 2-18 carbon atoms in the chain.

3. A metal carbamate according to claim 1, wherein the alkyl groups R₁ and R₂ each comprise 2-7 carbon atoms in the chain.

4. A metal carbamate according to claim 1, wherein the alkyl groups R₁ and R₂ are selected from the group comprising an ethyl, propyl, butyl, di-2-methylpropyl, di-2-methylpropyl, di-3-methylbutyl, di-n-pentyl, 2-methoxyethyl, 2-ethoxyethyl or a 2,2,2-trifluoroethyl group.

5. A metal carbamate according to claim 1, wherein the alkyl groups comprise heteroatoms.

6. A metal carbamate according to claim 1, wherein the alkyl groups comprise oxygen atoms.

7. A process for preparing metal carbamates of the general formula (I) by reacting
a) tolylenediamine with
b) an alkyl carbonate of the general formula (II) 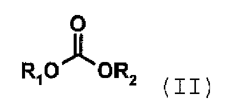 where R₁ and R₂ are each as defined above and
c) a metal compound of the general formula (III)
M(R₃)ₙ
where
M is an alkali metal,
R₃ is the same as R₁ and R₂ or is an amide or an alkylsilazide and
n is equal to 1.

8. A process for preparing methylenediphenyldiurethane, which comprises reacting a compound of the general formula (I) with water.

## Revendications

1. Carbamates métalliques de formule générale (I) 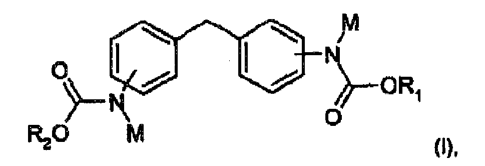 R₁ et R₂, identiques ou différents, signifiant un groupe alkyle comprenant 1 à 18 atomes de carbone, et M signifiant un ion de métal alcalin.

2. Carbamates métalliques selon la revendication 1, **caractérisés en ce que** les groupes alkyle R₁ et R₂ contiennent 2-18 atomes de carbone dans la chaîne.

3. Carbamates métalliques selon la revendication 1, **caractérisés en ce que** les groupes alkyle R₁ et R₂ contiennent 2-7 atomes de carbone dans la chaîne.

4. Carbamates métalliques selon la revendication 1, **caractérisés en ce que** les groupes alkyle R₁ et R₂ sont choisis dans le groupe contenant le groupe éthyle, propyle, butyle, di-2-méthylpropyle, di-2-méthylpropyle, di-3-méthylbutyle, di-n-pentyle, 2-méthoxyéthyle, 2-éthoxyéthyle ou 2,2,2-trifluoroéthyle.

5. Carbamates métalliques selon la revendication 1, **caractérisés en ce que** les groupes alkyle contiennent des hétéroatomes.

6. Carbamates métalliques selon la revendication 1, **caractérisés en ce que** les groupes alkyle contiennent des atomes d'oxygène.

7. Procédé pour la préparation de carbamates métalliques de formule générale (I) par transformation de
a) toluylènediamine avec
b) un alkylcarbonate de formule générale (II), 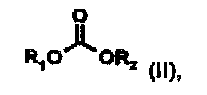 R₁ et R₂ ayant la signification susmentionnée, et
c) un composé métallique de formule générale (III)
M (R₃) ₙ
où
M signifie un métal alcalin,
R₃ est identique à R₁ et R₂ ou représente un amide ou un alkylsilazide et
n vaut 1.

8. Procédé pour la préparation de méthylènediphényldiuréthane, **caractérisé en ce qu'**un composé de formule générale (I) est transformé avec de l'eau.
